# EUROPEAN PATENT APPLICATION

(11) **EP 2 647 357 A1**
(43) Date of publication of application: **09.10.2013**
(21) Application number: 11845843.9
(22) Date of filing: 11.11.2011
(51) Int. Cl.: A61F 2/84

(54) **MEDICAL CATHETER DEVICE**

(30) Priority: 29.11.2010 JP 2010264699
(71) Applicant: Kyoto Medical Planning Co., Ltd., Kyoto-shi, Kyoto 607-8035 (JP)
(72) Inventor: IGAKI, Keiji, Kyoto-shi Kyoto 607-8035 (JP); YAMADA, Hirokazu, Kyoto-shi Kyoto 607-8035 (JP)
(74) Representative: Müller - Hoffmann & Partner
(86) International application number: PCT/JP2011/006328
(87) International publication number: WO 2012/073434

(57) **Abstract**

This invention is a catheter apparatus comprising a catheter (2) and a sheath (1) into which the catheter is inserted. The catheter has a distal end to which an insertion guiding member (5) is attached, provided at its distal end with a balloon (3) to expand a vascular stent (4), and provided with an expansion medium supplying channel for supplying an expansion medium to inflate the balloon and with a guide wire insertion channel for inserting a guide wire. The sheath into which the catheter is inserted is operated to move relative to the catheter between the section where it is covering the balloon attaching the vascular stent and the section where it is supposed to expose the vascular stent attached to the balloon. Between the sheath and the catheter, a first movement restrictor (35) protruding from the distal end of the catheter for restricting movement overpassing the insertion guiding member and a second movement restrictor (40) for restricting protruding-out length of the end portion of the catheter are provided, thereby restricting movable position of the sheath relative to the catheter such that excessive movement of the sheath is restricted, injuring of a blood vessel is prevented and the stent is surely implanted to a desired site in a blood vessel.

## Description

### Technical field

The present invention relates to a medical catheter apparatus useful for implanting a stent into a vessel in a living body.

### Background Art

Heretofore, when stenosis occurs in a vessel of a living body, such as a coronary artery, percutaneous transluminal angioplasty (PTA) is performed in which the stenosed portion in the vessel is expanded to improve the blood flow using a medical balloon catheter.

The site once stenosed, however, is known to have high possibility of restenosis or acute occlusion due to intimal dissection even after PTA. To prevent such acute occlusion or restenosis, a tubular shaped stent is implanted at the site following PTA. The stent used herein is in a contracted state when introduced into a blood vessel, and subsequently expanded in diameter so as to be deployed at the intended site scaffolding the vessel wall from its inside.

The stent to be implanted in a blood vessel is inserted and transported to the intended site in the vessel by using a catheter having a balloon which can be dilated with expansion medium supplied thereto. Specifically, the contracted stent is mounted onto the balloon provided at the distal portion of the catheter to be inserted into the blood vessel and transported to the intended site together with the balloon. With the balloon inflation by supplying the expansion medium into it, the stent is expanded in diameter, and consequently deployed at the lesion site. The once expanded stent keeps its expanded state even after the balloon is deflated by removal of the expansion medium, thereby scaffolding the implanted site radially to allow fluid path for humor such as blood in the vessel lumen.

Implantation of the stent using the above-mentioned catheter follows the procedure below.

First, prior to the catheter, a guide wire inserted into the catheter is inserted into a blood vessel, passing through a stenosed portion in the blood vessel. Next, the catheter is inserted into the blood vessel with the guidance of the guide wire to locate the balloon on which the stent is mounted at the stenosed portion which is the site for stent implantation. When the balloon is located at the stenosed portion along with the stent, by using an indeflator or the like, expansion medium is supplied to the balloon via an expansion medium supplying channel provided in the catheter, to inflate the balloon so that the stent is expanded in diameter to administer a treatment to expand the stenosed portion in the blood vessel. After the inflation of the balloon and the expansion treatment of the stenosed portion in the blood vessel, the expansion medium having supplied to the balloon is drawn for decompression and contraction of the balloon. At this time, the stent previously expanded in diameter keeps the expanded state, is disengaged from the balloon contracted in diameter and remained at the stenosed portion in the blood vessel for keeping the state scaffolding the inner wall of the blood vessel. Finally, the catheter is removed from the body and the stenting is completed.

When implanting a stent into a vessel such as a blood vessel by using a balloon catheter, the stent mounted on the balloon might be dislocated from a correct mounting position or disengaged from the balloon during the insertion operation. In order to solve this problem, the present inventors has proposed a catheter apparatus for providing a stent in which a stent mounted on a balloon is retracted into a protective sheath along with the balloon before inserted into a vessel (WO2004/013450 A1).

In this sort of catheter apparatus, the stent retracted in the sheath is inserted into a blood vessel, and when the stent arrives at an implantation site, pulling and moving the sheath against the catheter make the stent outwardly protruding from a distal end of the sheath. By supplying expansion medium to a balloon, the stent released from the state covered by the sheath is expanded along with the balloon inflation, thus completing the stent implantation at the implantation site in the blood vessel.

### Citation List

### Patent Literature

PTL 1: WO2004/103450

### Summary of the Invention

### Technical Problem

In a usual catheter apparatus, a load applied thereto would prohibit further insertion when, for example, a distal end of a catheter abuts on an inner wall of a blood vessel during insertion process. In this situation, especially in the case of a catheter apparatus having a sheath covering a stent, enforced insertion in the blood vessel would only moves the sheath toward the distal direction of the catheter, resulting in that the sheath overpasses an insertion guiding member provided at a distal end of the catheter. This insertion guiding member provided at the distal end of the catheter is provided in order to assist smooth insertion of the catheter apparatus into a blood vessel and to prevent the sheath from protruding out from the distal end, the proximal end of the insertion guiding member having an outer diameter approximately equal to the outer diameter of the sheath. The distal side of the insertion guiding member is tapered off toward its end.

If the sheath overpasses the insertion guiding member and reaches in front of the catheter, the outer diameter of the distal end of the sheath might be expanded, resulting in a possibility of injuring an inner wall of the blood vessel. A sharp corner of the sheath tip also has a risk of injuring an inner wall of the blood vessel.

Furthermore, if the sheath tip protrudes from the catheter tip, the insertion guiding member might be engaged with the sheath, prohibiting smooth movement of the sheath against the catheter. In this case, a problem would occur wherein the stent held by the catheter cannot be protruded from the sheath and be implanted to a desired implantation site.

A technical object of the present invention is therefore to provide a medical catheter apparatus which allows smooth and safe implantation of a stent at a desired implantation site of a vessel while protecting a vessel such as a blood vessel.

Another technical object of the present invention is to provide a medical catheter apparatus which allows efficient implantation of a stent into a vessel of a living body while protecting the stent mounted on a balloon provided on a catheter.

Further technical object of the present invention is to provide a medical catheter apparatus which allows rapid replacement of catheters and efficient implantation of a plurality of stents into a vessel of a living body.

### Solution of Problem

To achieve the above-mentioned technical objects, the present invention provides a catheter apparatus comprising a catheter and a sheath into which the catheter is inserted. The catheter used in this catheter apparatus has a distal end to which an insertion guiding member is attached. The catheter is provided at its distal end with a balloon to be inflated by supplying an expansion medium to expand a vascular stent mounted on an outer periphery of the balloon, and provided with an expansion medium supplying channel extending from the proximal end to the distal end of the catheter for supplying the expansion medium to inflate the balloon and with a guide wire insertion channel extending at least from the distal end to the middle portion of the catheter for inserting a guide wire. The sheath into which the catheter is inserted covers the outer periphery of the catheter from its distal side where the balloon is positioned through its proximal side, and be operated to move relative to the catheter between the section where it is covering the balloon attaching the vascular stent and the section where it is supposed to expose the vascular stent attached to the balloon. Between the sheath and the catheter, a first movement restrictor protruding from the distal end of the catheter for restricting movement overpassing the insertion guiding member and a second movement restrictor for restricting protruding-out length of the end portion of the catheter are provided.

At a proximal portion of the sheath, a connecting member is provided having a catheter drawing-out port for drawing-out the catheter inserted into the sheath to the distal end of the sheath, and the first movement restrictor is provided inside the catheter drawing-out port.

The first movement restrictor comprises an abutting stopper formed inside the catheter drawing-out port and a movement restriction member provided at the catheter for abutting the abutting stopper to restrict relative movement between the sheath and the catheter.

The insertion guiding member attached to the distal end of the catheter includes a proximal portion having a diameter larger than an inner diameter of the sheath and is formed as a shape tapered off toward its distal end. The movement of the sheath against the catheter is restricted by the first movement restrictor, thereby preventing the distal portion of the sheath from overriding the insertion guiding member and being expanded in diameter.

It is desirable to provide a catheter fixing mechanism at a proximal portion of the sheath for clamping and supporting the sheath against the catheter, thereby fixing the movement of the sheath against the catheter.

The catheter apparatus according to the present invention further comprises a guide wire leading-out opening provided at a middle portion of the catheter for leading out the guide wire inserted from the distal end of the catheter toward the side direction of the catheter and a guide wire drawing-out opening provided at a middle portion of the sheath for drawing out the guide wire led out from the catheter out of the sheath. The catheter apparatus further comprises a guide wire drawing-out guiding mechanism provided between the guide wire leading-out opening and the guide wire drawing-out opening for connecting the guide wire leading-out opening and the guide wire drawing-out opening and for guiding the guide wire led out from the guide wire leading-out opening to the guide wire drawing-out opening. The guide wire drawing-out guiding mechanism moves in accordance with the relative movement between the sheath and the catheter, with its length extending from the guide wire leading-out opening to the guide wire drawing-out opening variable.

The guide wire drawing-out guiding mechanism comprises a first tubular member, the proximal end of which is connected with the guide wire leading-out opening, extending along the outer surface of the catheter toward the guide wire drawing-out opening formed on the sheath, and a second tubular member, the proximal end of which is connected with the guide wire drawing-out opening formed on the sheath 1, extending along the inner surface of the sheath toward the guide wire leading-out opening formed on the catheter, and the first tubular member and the second tubular member are movably connected by fitting their end portions.

More particularly, the first tubular member constituting the guide wire drawing-out guiding mechanism is formed as a tubular member having a length sufficient to be inserted into the second tubular member by an overlap length L2 longer than a migration length L1 of the sheath to outwardly expose the balloon provided at the distal portion of the catheter when the sheath is pulled with respect to the catheter.

The catheter apparatus further comprises a second movement restrictor provided between the sheath and the catheter for restricting protruding-out length of the end portion of the catheter to which the balloon is attached.

The second movement restrictor comprises a stopping step formed in the catheter drawing-out port and an abutting stopper provided on an outer periphery of the catheter for abutting the stopping step to restrict relative movement between the sheath and the catheter.

### Advantageous Effects of Invention

In a medical catheter apparatus according to the present invention, movement of a sheath, into which a catheter is inserted and covering a stent mounted on a balloon provided at a distal portion of the catheter, toward the distal end of the catheter is restricted by a first movement restrictor, thereby preventing that only the sheath moves toward the distal end of the catheter and overpasses an insertion guiding member provided at a distal end of the catheter during insertion process into a vessel such as a blood vessel. By restricting the movement of the sheath to prevent the distal portion of the sheath from overpassing the insertion guiding member and protruding out of the distal end of the catheter, an accident wherein the distal end of the sheath injures an inner wall of a blood vessel during an insertion operation into a vessel can be surely avoided.

By locating the first movement restrictor inside the catheter drawing-out port provided in a connecting member attached to the proximal portion of the sheath, and to which a liquid such as a saline solution is supplied, faulty release of the movement restriction can be avoided and the movement of the sheath against the catheter is surely restricted.

Furthermore, the first movement restrictor, comprising an abutting stopper formed inside the catheter drawing-out port and a movement restriction member provided at the catheter for abutting the abutting stopper to restrict relative movement between the sheath and the catheter, not only has a simple structure but also achieves a certain restriction of the movement of the sheath against the catheter.

In addition, the present invention can prevent the distal portion of the sheath from overpassing the insertion guiding member and being expanded in diameter, thereby surely preventing the distal portion of the sheath from injuring an inner wall of a blood vessel.

Furthermore, by providing a catheter fixing mechanism for fixing the movement of the sheath against the catheter, the sheath and the catheter are unified together and able to be inserted into a vessel while preventing protrusion of the sheath toward the distal end of the catheter, such that the stent can be safely and surely delivered and implanted to a lesion site in a vessel.

Moreover, the present invention employs, in a catheter apparatus, a sheath covering a catheter provided with a balloon attaching a vascular stent and a guide wire drawing-out guiding mechanism for leading out a guide wire toward the side direction from the middle portion of the catheter, thereby making insertion length of the guide wire inserted into the catheter shorter, thus achieving an easy and quick replacement of catheters and a safe insertion operation into a vessel.

By employing a first tubular member connected to the catheter and a second tubular member connected to the sheath as the guide wire drawing-out guiding mechanism, even when the catheter is moved relative to the sheath, the guide wire can be inserted into the first and second tubular members and drew out from the sheath, such that the guide wire inserted into the catheter can be drew out from middle portion of the catheter and surely led out from guide wire leading-out opening provided at the sheath, while preventing the sheath from protruding out from the distal end of the catheter, and enabling the stent safely and surely delivered to a lesion site in a vessel and implanted therein.

Furthermore, by forming the first tubular member constituting the guide wire drawing-out guiding mechanism as a tubular member having a length sufficient to be inserted into the second tubular member by an overlap length L2 longer than a migration length L1 of the sheath to outwardly expose the balloon provided at the distal portion of the catheter when the sheath is pulled with respect to the catheter, disengagement between the first and second tubular member can be always prevented and protrusion of the sheath from the distal end of the catheter can be also prevented even when the guide wire inserted into the catheter is drew out from the sheath.

In addition, since the catheter apparatus according to the present invention restricts protruding-out length of the end portion of the catheter to which the balloon is attached by using the second movement restrictor, the stent mounted on the balloon can be surely exposed out of the sheath and be safely and surely implanted in a lesion site in a vessel and implanted therein.

Similarly to the first movement restrictor, the second movement restrictor also comprises an abutting stopper formed inside the catheter drawing-out port and a movement restriction member provided at the catheter for abutting the abutting stopper to restrict relative movement between the sheath and the catheter, thereby achieving a certain restriction of the movement of the sheath against the catheter while simplifying the structure thereof.

### Brief Description of the Drawings

Figure 1 is a side view of a medical catheter apparatus according to the present invention showing its appearance.
Figure 2 is a cross-sectional view showing a catheter and a sheath of a catheter apparatus according to the present invention wherein the balloon on the distal portion of the catheter and the stent mounted on the balloon are covered with the sheath.
Figure 3 is a side view of a catheter constituting a catheter apparatus according to the present invention.
Figure 4 is a cross-sectional view taken along line IV-IV of figure 3.
Figure 5 is a side view of the first tubular member constituting the guide wire drawing-out guiding mechanism connected with the catheter.
Figure 6 is a perspective view of the guide wire drawing-out guiding mechanism wherein the balloon on the distal portion of the catheter and the stent mounted on the balloon are covered with the sheath.
Figure 7 is a perspective view of the guide wire drawing-out guiding mechanism wherein the balloon on the distal portion of the catheter and the stent mounted on the balloon are exposed outwardly.
Figure 8 is a cross-sectional view of a restrictor for restricting movement of the sheath against the catheter showing a state in which the forward movement of the sheath toward the distal end of the catheter is restricted.
Figure 9 is a cross-sectional side view showing a state wherein the balloon on the distal portion of the catheter and the stent mounted on the balloon are exposed outwardly to make it possible to expand the stent on the balloon.
Figure 10 is a cross-sectional view of a restrictor for restricting movement of the sheath against the catheter showing a state in which the sheath is moved relative to the catheter to protrude the stent mounted on the distal portion of the catheter 2 from the distal end of the sheath.

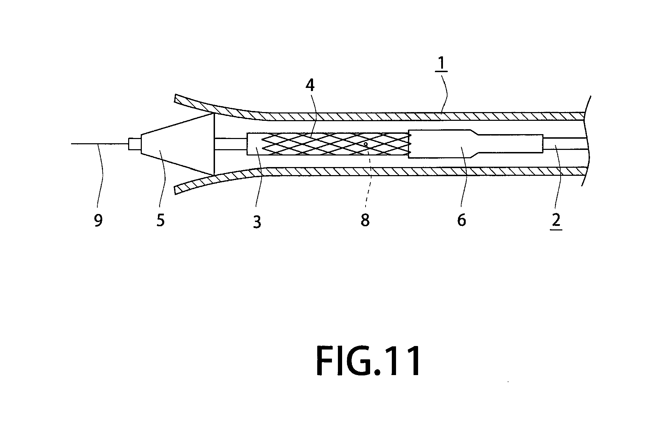

Figure 11 is a cross-sectional view showing a state in which the distal portion of the sheath protrudes from the distal end of the catheter.

### Description of Embodiments

The present invention is described hereinafter based on illustrative embodiments of catheter apparatus useful to percutaneous transluminal angioplasty (PTA) in which a stenosed portion in a vessel of a living body, such as a blood vessel, is expanded to improve blood flow.

This embodiment explain the present invention by illustrating an example applied to a monorail catheter in which a guide wire is led out toward the side direction from the middle portion of the catheter.

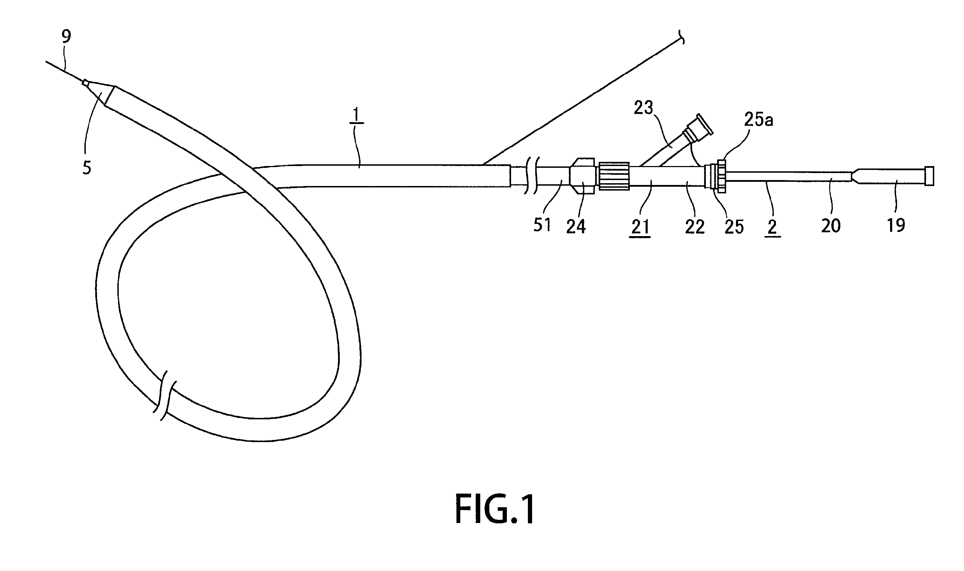

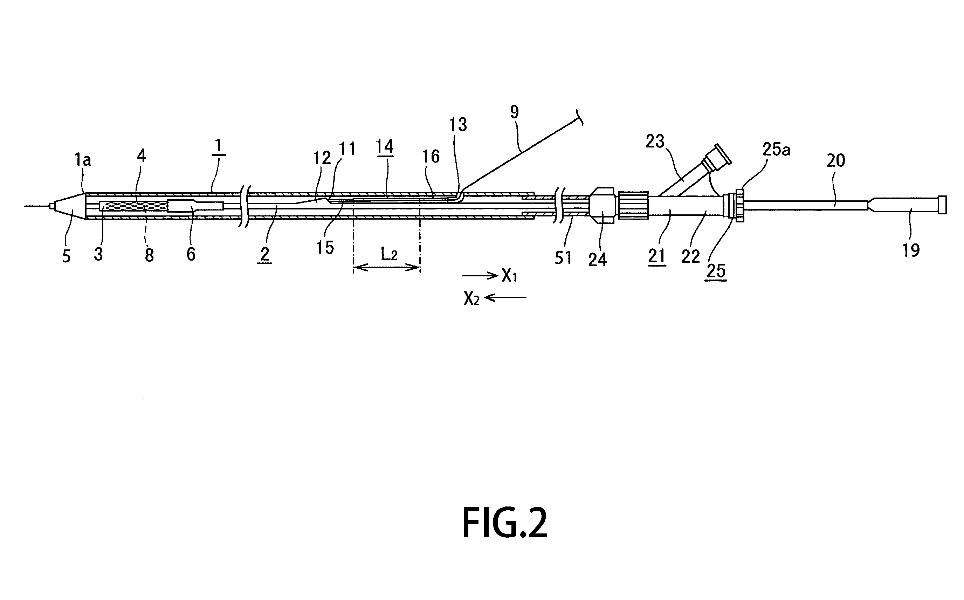

The catheter apparatus according to the present invention includes a protective sheath 1 and a catheter 2 contained in the sheath 1 with movability to back and forth, as shown in Fig.1 and 2.

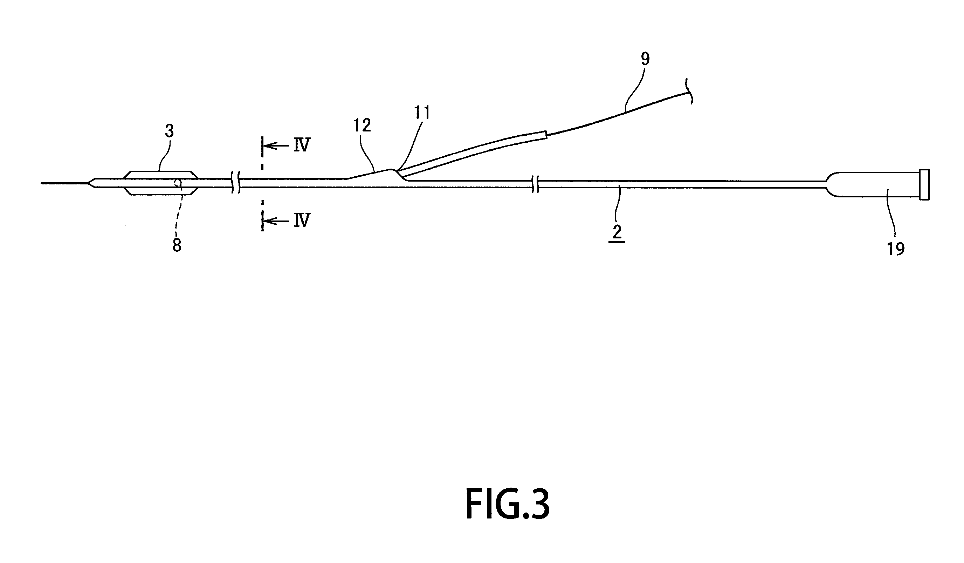

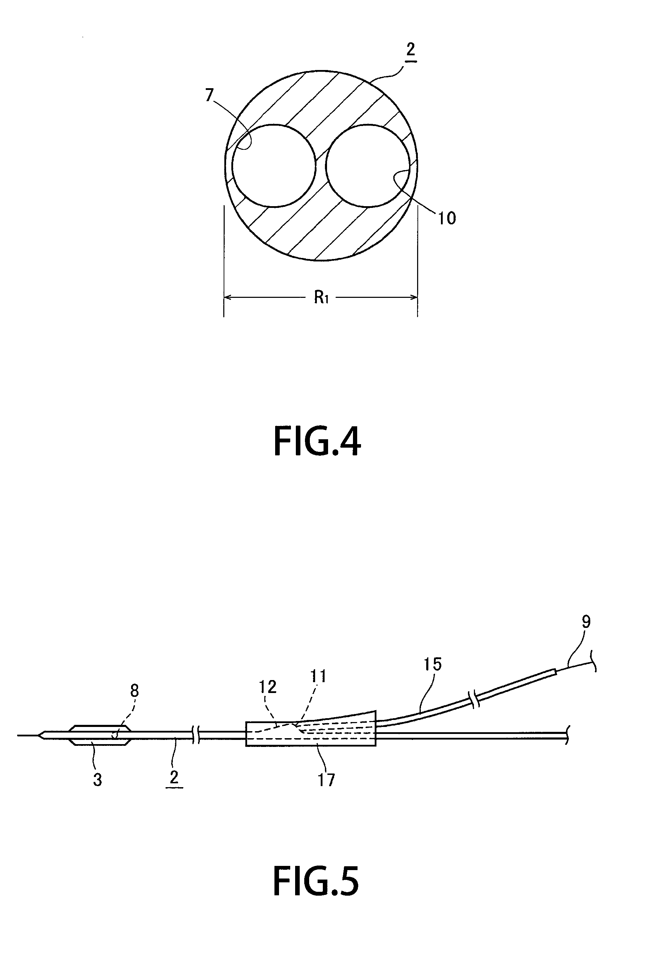

The catheter 2 constituting this catheter apparatus is shaped as an elongated tubular member, as shown in Figures 3 and 4, having a diameter of 1 to 2mm and an entire length of 70 to 150cm. This catheter 2 is shaped such that it can be inserted with easy deformation in accordance with a curved or circuitous vessel in a living body, such as a blood vessel. It should be noted that a conventionally and broadly used type of vascular stent may be employed as the catheter 2. The sheath 1 is also made of a material capable of flexible deformation in the longitudinal direction. In this embodiment, the sheath 1 is made of a polyamide resin.

At the distal end of the catheter 2, a balloon 3 which is inflated by an expansion medium such as a contrast medium is provided, as shown in figures 2 and 3. On the outer periphery of this balloon 3, a stent 4 to be deployed in the desired site in the blood vessel is mounted. This stent 4 is formed by using, for example, a strand of a biodegradable polymer into a cylindrical shape with a channel therein extending from its one end to the other. This cylindrically shaped stent 4 is mounted on the outer periphery of the balloon 3 and will be expanded in diameter along with the inflation of the balloon 3.

At the distal end of the catheter 2, an insertion guiding member 5 is attached. The insertion guiding member 5 is provided for assisting smooth insertion of the catheter apparatus into a vessel such as a blood vessel and for preventing the sheath 1 from protruding out from the distal end of the catheter 2 and the diameter of the proximal portion of the insertion guiding member is nearly equal to the outer diameter of the sheath 1. It should be noted that, at central portion of the insertion guiding member 5, a through hole for inserting a guide wire 9 is provided as described below.

The proximal side of the stent 4 mounted on the balloon 3 is held by a stent holding member 6. The stent holding member 6 is provided so as to prevent the stent 4 from being displaced relative to the balloon 3 during stent expansion, and to ensure reliable expansion of the stent 4 in accordance with the inflation of the balloon 3.

The catheter 2 is provided with an expansion medium supplying channel 7 through which an expansion medium flows for expanding the balloon 3 on the distal end of the catheter 2 (see figure 4). The expansion medium supplying channel 7 is formed as a continuous channel which is continuously extending from the proximal end of the catheter 2 to the distal end thereof on which the balloon 3 is provided. As shown in figure 3, a through hole 8 connected with the expansion medium supplying channel 7 is opened in the portion of the catheter 2 on which the balloon 3 is attached. Via the through hole 8, expansion medium supplied to the expansion medium supplying channel 7 flows into the balloon 3 to fill the balloon 3, or the expansion medium filling the balloon 3 is evacuated.

The section extending from the distal end to the middle portion of the catheter 2 is provided with a guide wire insertion channel 10 into which a guide wire 9 for guiding the insertion direction of the catheter 2 is inserted. Thus, as shown in figure 4, the section extending from the distal end to the middle portion of the catheter 2 is formed as a two-channel catheter wherein two channels, the expansion medium supplying channel 7 and the guide wire insertion channel 10, are independently and parallelly configured.

The guide wire insertion channel 10 is formed with its distal open end being exposed at the distal end of the catheter 2 and its proximal open end being exposed at a side surface of the middle portion of the catheter 2. The proximal open end is used for a guide wire leading-out opening 11 for leading out the guide wire 9 out of the catheter 2. As shown in figure 3, the portion of the catheter 2 having the guide wire leading-out opening 11 is formed as a bulge 12 which is gradually bulged toward side direction to make a slope.

It should be noted that the guide wire insertion channel 10 is formed along a length extending from the distal end of the catheter 2 to a point of 10 to 40cm therefrom. Thus, the guide wire leading-out opening 11 is provided at a position 10 to 40cm apart from the distal end of the catheter 2.

In addition, as shown in figure 2, there is provided at the middle portion of the sheath 1 into which the catheter 2 is inserted, a guide wire drawing-out opening 13 for outwardly drawing out the guide wire 9 which is led out from the guide wire leading-out opening 11.

Between the guide wire leading-out opening 11 formed on the catheter 2 and the guide wire drawing-out opening 13 formed on the sheath 1, a guide wire drawing-out guiding mechanism 14 connecting the guide wire leading-out opening 11 and the guide wire drawing-out opening 13 is provided for guiding the guide wire 9 led out from the guide wire leading-out opening 11 to the guide wire drawing-out opening 13.

The guide wire drawing-out guiding mechanism 14 moves back and forth in accordance with the relative movement between the sheath 1 and the catheter 2, with its length extending from the guide wire leading-out opening 11 to the guide wire drawing-out opening 13 variable. By changing the length of this guide wire drawing-out guiding mechanism 14 in accordance with the change of the distance between the guide wire leading-out opening 11 and the guide wire drawing-out opening 13 resulted from the relative movement between the sheath 1 and the catheter 2, straight state of the guide wire 9 drew out from the catheter 2 to the sheath 1 can be kept without warping or bending the guide wire 9.

In this embodiment, as shown in figure 2, the guide wire drawing-out guiding mechanism 14 is constituted of a first tubular member 15, the proximal end of which is connected with the guide wire leading-out opening 11 formed on the catheter 2, extending along the outer surface of the catheter 2 toward the guide wire drawing-out opening 13 formed on the sheath 1, and a second tubular member 16, the proximal end of which is connected with the guide wire drawing-out opening 13 formed on the sheath 1, extending along the inner surface of the sheath toward the guide wire leading-out opening 11 formed on the catheter 2.

The first and second tubular members 15 and 16 are formed as a hollow tubular member having an inner diameter sufficient to accommodate the guide wire 9. The tubular members 15 and 16 used in this embodiment are preferably formed of a tube made of a lubricous synthetic resin with little friction so that the guide wire 9 formed of a metal wire having a diameter of 0.3 to 0.6 mm can be smoothly inserted. In this embodiment, the first and second tubular members 15 and 16 are formed of a polyimide synthetic resin.

In addition, as shown in figure 5, the first tubular member 15 is connected with the catheter 2 by fitting an open end at one side with the guide wire leading-out opening 11 and is extended along the outer surface of the catheter 2 toward the proximal end of the catheter. It should be noted that the proximal end of the first tubular member 15 is clamped to the catheter 2 by a ring-shaped member 17 made of a synthetic resin fitted on the outer periphery of the catheter 2 so as not to be disengaged from the catheter 2, as shown in figure 5.

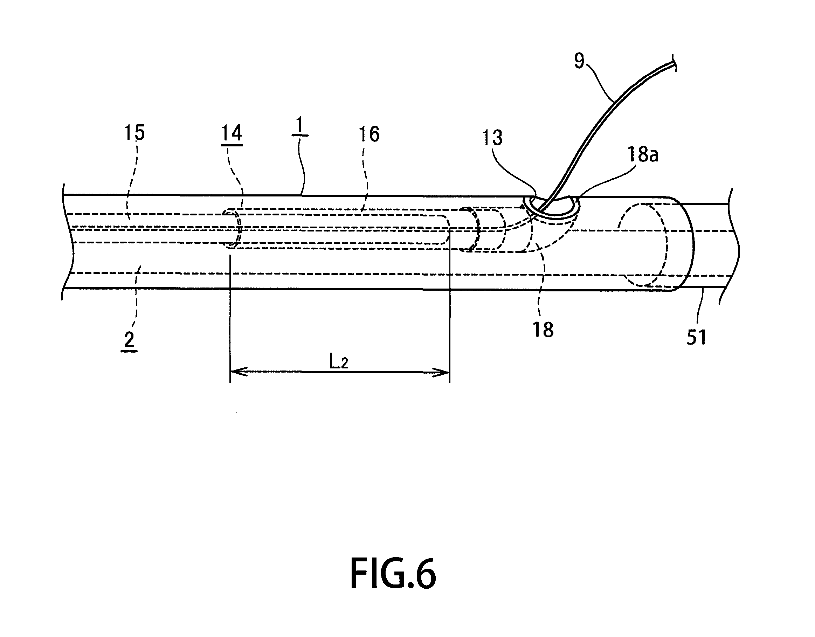

In addition, the second tubular member 16 is connected with the sheath 1 by joining the proximal end surface to the periphery of the guide wire drawing-out opening 13, as shown in figure 6. Specifically, the second tubular member 16 is extended along the inner surface of the sheath 1 with the distal end extending toward the guide wire leading-out opening 11.

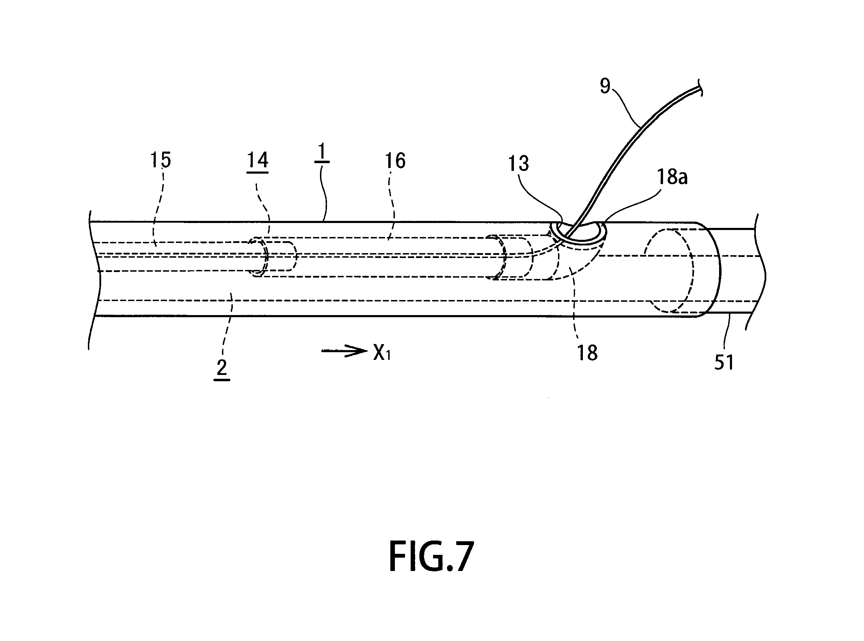

In this embodiment, as shown in figures 6 and 7, the second tubular member 16 is connected with the sheath 1 via a connecting member 18 made of a synthetic resin. It should be noted that the connecting member 18 is connected with the second tubular member 16 by fitting its one end with the proximal end of the second tubular member 16. The connecting member 18 is connected with the inner surface of the sheath 1 by joining the proximal end surface 18a to the periphery of the guide wire drawing-out opening 13. The second tubular member 16 is connected with the sheath 1 by joining the connecting member 18 connected to the proximal end to the sheath 1.

The joining of the connecting member 18 to the sheath 1 is conducted by using a thermal welding wherein portions of the connecting member 18 and sheath 1 are welded together. Specifically, the whole perimeter of the proximal end surface 18a of the connecting member 18 is joined to the whole perimeter of the guide wire drawing-out opening 13. Consequently, the guide wire drawing-out opening 13 is sealed, thus preventing leakage of a liquid such as a saline solution injected into the sheath 1 and completely removing air from the catheter apparatus.

As shown in figures 1, 2 and 3, an indeflation port 19 connected to the expansion medium supplying channel 7 is provided at the proximal end of the catheter 2 to be inserted into the sheath 1. Although not shown, an indeflator is connected with this indeflation port 19 to supply expansion medium to inflate the balloon 3. The expansion medium provided from the indeflator enters into the indeflation port 19, flows through the expansion medium supplying channel 7, and is supplied to the balloon 3 via a through hole 8 opened at a portion of the catheter 2 on which the balloon 3 is attached to inflate the balloon 3. As the balloon 3 is inflated, the stent 4 mounted on the outer periphery of this balloon 3 is expanded in diameter in accordance with the inflation of the balloon 3.

It should be noted that a warping restriction member 20 for restricting the warping of the catheter 2 is attached to the proximal end of the catheter 2 to which the indeflation port 19 is connected. The warping restriction member 20 is formed from a material having a high stiffness such as aluminum or stainless steel as a hollow tube which cannot be easily deformed or warped, and attached to the catheter 2 by inserting the outer surface of the catheter 2 into the warping restriction member 20.

As stated above, the catheter 2, on the distal end of which has a balloon 3 on which the stent 4 is mounted, is inserted into the sheath 1 movably back and forth and a connecting member 21 is attached to the proximal end of the sheath, from which the proximal end of the catheter 2 is drew out. This connecting member 21 is provided with a catheter drawing-out port 22 from which the catheter 2 inserted into the sheath 1 protrudes and an indeflation port 23 for supplying a liquid such as a saline solution to remove air in the sheath 1.

The connecting member 21 is attached to the sheath 1 via a connecting hub 24 attached to the proximal portion of the sheath 1. The proximal portion of the catheter port 22 provided in the connecting member 21 includes a clamp fixing mechanism 25 which provides a fixing mean for fixing the relative movement between the sheath 1 and the catheter 2 protruding from the proximal end of the sheath 1.

In addition, in a catheter apparatus according to the present invention, a tubular connection assist member 51 having a stiffness is connected to the proximal portion of the sheath 1. This connection assist member 51, provided for preventing flexible deformation of the proximal portion of the sheath 1, is formed of a tubular member made of a metal such as stainless steel or aluminum.

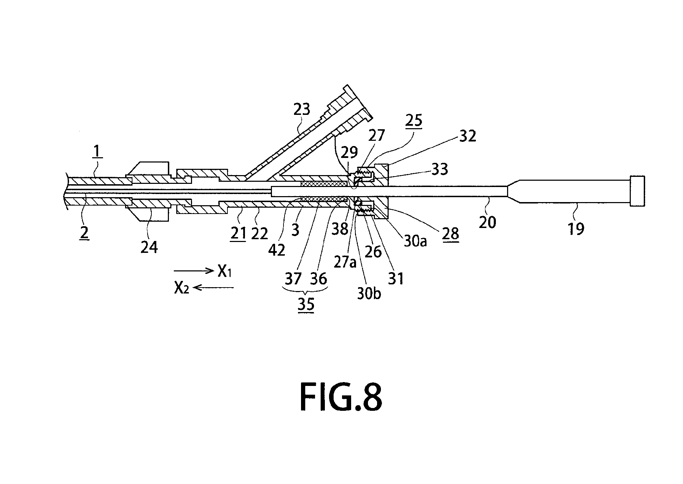

As shown in figure 8, this clamp fixing mechanism 25 comprises a catheter compressing member 26 formed of an elastic material such as rubber through which the catheter 2 is inserted, a compressing member holder 27 for accommodating and holding the catheter compressing member 26, and a compressing and fixing tool 28 for compressing and fixing the catheter compressing member 26.

The catheter compressing member 26 is formed as a ring shape, with its center portion being a catheter insertion hole 29 through which the catheter 2 is inserted. The compressing member holder 27 is formed uniformly with the catheter drawing-out port 22 by expanding the proximal end of the catheter drawing-out port 22 in diameter. The catheter compressing member 26 is accommodated in the compressing member holder 27 such that the catheter compressing member 26 abuts the bottom surface 27a of the compressing member holder 27. The compressing and fixing tool 28 is movably attached to the compressing member holder 27, by screwing a tubular fitting portion 31 having a screw thread 30a on its inner surface into the compressing member holder 27 having a screw thread 30b on its outer surface. At the proximal end of the fitting portion 31, a rotating operation member 32 is provided for rotating the compressing and fixing tool 28. At the center portion of this compressing and fixing tool 28, a compressing operation member 33 is provided for compressing the catheter compressing member 26 accommodated in the compressing member holder 27 such that the catheter compressing member 26 tightly contacts with the catheter 2 inserted into this catheter compressing member 26. The compressing operation member 33 is located on the inner surface of the fitting portion 31 and formed as a tubular shape coaxially with the fitting portion 31. Through this compressing operation member 33, the catheter 2 is inserted. By screwing the compressing and fixing tool 28 into the compressing member holder 27, the compressing operation member 33 protrudes into the compressing member holder 27 to compress the catheter compressing member 26.

By pushing the catheter compressing member 26 with the compressing operation member 33 onto the bottom surface 27a of the compressing member holder 27, the expansion-restricted outer diameter of the catheter compressing member 26 is reduced, thus reducing the outer diameter of the catheter insertion hole 29 and clamping the catheter 2. Clamping the catheter 2 with the catheter compressing member 26 restricts movement of the catheter 2 against the sheath 1 to fix the insertion position of the catheter 2 against the sheath 1, such that the relative movement between the sheath 1 and the catheter 2 is restricted.

Moreover, by clamping the catheter 2 with the catheter compressing member 26, the inside space of the compressing member holder 27 is sealed. This sealing of the proximal end of the connecting member 21 prevents leakage from the catheter drawing-out port 22 of a liquid such as a saline solution supplied from the indeflation port 23 and make it possible to supply the liquid to sheath 1.

It should be noted that after fixing the insertion position of the catheter 2 against the sheath 1, releasing the clamping of the clamp fixing mechanism 25 makes relative movement against the sheath possible. This release of the fixing of the catheter 2 with the clamp fixing mechanism 25 is executed by rotating the compressing and fixing tool 28, screwing out the compressing operation member 33 from the catheter compressing member 26 and releasing the compression of the catheter compressing member 26.

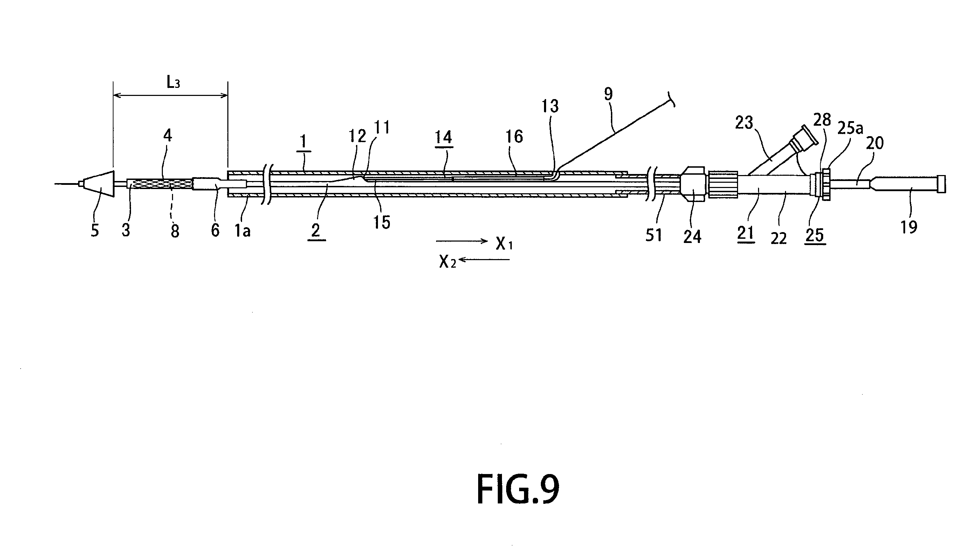

In the catheter apparatus according to the present invention constituting a monorail catheter, the first tubular member 15 constituting the guide wire drawing-out guiding mechanism 14 is formed as a tubular member having an overlap length L2 longer than the length L1 of the distal section of the catheter 2 which is exposed out of the sheath 1 as shown in figure 9, when the sheath 1 is pulled with respect to the catheter in the direction of arrow X1 shown in figure 7 toward the operator (see also figures 2, 6 and 10). This constitution of the first tubular member 15 keeps the connection between the first and second tubular members 15 and 16 even when the sheath 1 is pulled with respect to the catheter 2 in the direction of arrow X1 shown in figure 9 toward the operator to remove the cover and to expose the distal section of the catheter on which the balloon 3 is provided. Consequently, even when the pulling operation of the sheath 1 against the catheter 2 changes the distance between the guide wire leading-out opening 11 and the guide wire drawing-out opening 13, the connection between the first and second tubular members 15 and 16 is guaranteed, allowing the guide wire 9 to be surely drew out toward the side direction of the sheath 1.

The catheter apparatus according to the present invention is provided with a first movement restrictor 35 for preventing the sheath 1 from being pushed toward the direction of arrow X2 shown in figure 2 against the catheter 2 due to a misoperation which might occur when releasing the fixing with the clamp fixing mechanism 25 and then moving the sheath 1 against the catheter 2 in order for the stent 4 accommodated in the sheath 1 to protrude from the distal end of the catheter 2 together with the balloon 3, and for preventing the sheath 1 from overpassing the insertion guiding member 5 provided at the tip of the catheter 2 and moving to in front of the catheter 2 due to an enforced insertion under a situation wherein the distal end of the catheter 2 abuts on an inner wall of a blood vessel. The movement restriction member 35 is provided between the relatively movable sheath 1 and catheter 2. The first movement restrictor 35 is comprised of a first abutting stopper 36 provided on the sheath 1 and a movement restriction member 37 provided on the catheter 2.

In this embodiment, as shown in figure 8, the first abutting stopper 36 constituting the first movement restrictor 35 is constituted of a stopping step formed in the catheter drawing-out port 22 of the connecting member 21 attached to the proximal end of the sheath 1. This abutting stopper 36 is provided at the proximal end of the catheter drawing-out port 22 to which the clamp fixing mechanism 25 is connected. As shown in figure 8, the movement restriction member 37 is formed from a member attached on the outer surface of the catheter 2 which enlarges the outer diameter of the catheter 2. In this embodiment, it is formed by attaching a cylindrical member made of a synthetic resin to the outer peripheral potion of the catheter 2 which moves back and forth in the catheter drawing-out port 22. More particularly, the movement restriction member 37 is attached to the outer surface of the warping restriction member 20 connected to the proximal end of the catheter 2.

As shown in figure 8, the first movement restrictor 35 restricts the forward movement of the sheath 1 in the direction of arrow X2 shown in figure 8 toward the distal end of the catheter 2 when one abutting surface 38 located at the proximal end of the movement restriction member 37 abuts the first abutting stopper 36. At this time, as shown in figure 2, the distal end 1a of the sheath 1 abuts the proximal end of the insertion guiding member 5, thus keeping the stent 4 on the balloon 3 in the state accommodated in the sheath.

The movement restriction member 37 is, therefore, formed on the outer surface of the catheter 2 so that the distal end 1a of the sheath 1 abuts the proximal end of the insertion guiding member 5 when the first abutting stopper 36 on the sheath 1 abuts the one abutting surface 38.

Furthermore, the catheter apparatus according to the present invention is provided with a second movement restrictor 40 for restricting leading-out length of the sheath 1 against the catheter 2 to prevent release of connected state between the first and second tubular members 15 and 16. The second movement restrictor 40 is comprised of a second abutting stopper 41 provided on the sheath 1 and the movement restriction member 37 attached to the outer surface of the catheter 2. In this embodiment, the second abutting stopper 41 is formed by the proximal end surface of the hub 24 connecting the connecting member 21 to the sheath 1.

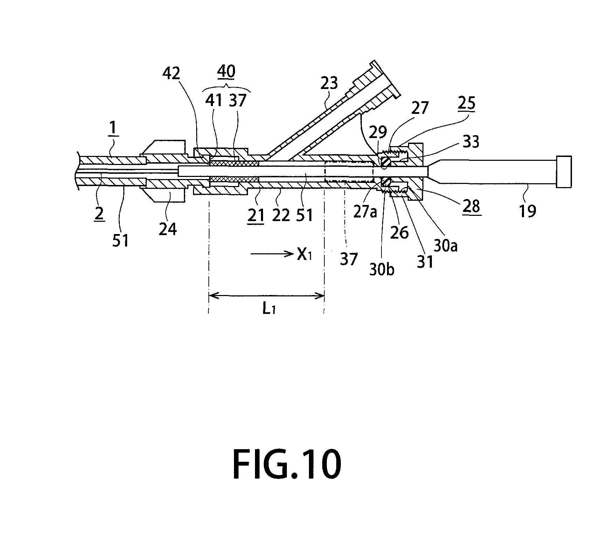

As shown in figure 10, movement of the sheath 1 against the catheter 2 in the direction of arrow X1 shown in figure 10 is restricted when the second abutting stopper 41 abuts the other abutting surface 42 located at the proximal end of the movement restriction member 37. The second movement restrictor 40 is located at a position to restrict the movement of the sheath 1 against the catheter 2 so as not to release the overlap between the first and second tubular members 15 and 16 when pulling operation of the sheath 1 against the catheter 2 in the pulling direction of arrow X1 shown in figure 10 causes the second tubular member 16 to move relative to the first tubular member 1 in the pulling direction of arrow X1 shown in figure 7.

That is, the second movement restrictor 40 is formed so that the migration length L1 of the sheath 1 against the catheter 2 in the direction of arrow X1 shown in figure 10 is shorter than the overlap length between the first and second tubular members 15 and 16. Since the migration length L1 of the sheath 1 against the catheter 2 corresponds to the length L3 by which the stent 4 mounted on the balloon 3 is exposed outwardly from the sheath 1 as shown in figure 9, the stent 4 can be exposed outwardly and be expanded in diameter with the inflation of the balloon 3. Particularly, since the second movement restrictor 40 restricts the position by which the stent 4 protrudes from the distal end of the sheath 1, the migration length of the sheath 1 against the catheter 2 is kept constant, the balloon 3 supported with the sheath is surely released, and thus the stent 4 can be expanded in diameter.

In this embodiment, the stent 4 mounted on the balloon 3 provided at the distal end of the catheter 2 protrudes from the distal end of the sheath 1 by moving the sheath 1 against the catheter 2 in the direction of arrow X1 shown in figure 9 until the second abutting stopper 41 abuts the other abutting surface 42 of the movement restriction member 37. The gap between the second abutting stopper 41 and the other abutting surface 42 of the movement restriction member 37, therefore, is formed to have a gap sufficient for the first abutting stopper 36 on the sheath 1 to be moved from the position abutting the abutting surface 38 of the movement restriction member 37 by the migration length L1, as shown in figure 10, which corresponds to the length L3 by which the stent 4 mounted on the balloon 3 is exposed outwardly from the sheath 1.

It should be noted that, in the catheter apparatus according to the present invention, by mounting the stent 4 in contracted state on the balloon 3 provided at the distal end of the catheter 2, the stent 4 is surely held by the sheath 1 such that dislocation or disengagement from the balloon 3 can be prevented.

Next, an example wherein the stent 4 is implanted in a blood vessel of a living body using a medical catheter apparatus according to the present invention will be explained.

Firstly, a catheter apparatus wherein the stent 4 is mounted on the balloon 3 is prepared. At this time, the stent 4 is mounted and held on the balloon 3 in a contracted state with a contracted diameter such that the stent 4 is retracted in the sheath 1. As shown in figure 2, the catheter 2 is retracted in the sheath 1 and fixed to the sheath 1 by the clamp fixing mechanism 25 so as to restrict the relative movement therebetween. At this time, as shown in figure 8, the first abutting stopper 36 constituting the first movement restrictor 35 abuts the abutting surface 38 of the movement restriction member 37 and thus restricts the forward movement of the sheath 1 in the direction of arrow X2 shown in figure 8 toward the distal end of the catheter 2.

Next, the proximal portion of the guide wire 9 having inserted into a stenosed portion in a blood vessel is inserted into the guide wire insertion channel 10 from the distal side of the catheter 2. By inserting the guide wire 9 further into the guide wire insertion channel 10, the guide wire 9 is led out from the guide wire leading-out opening 11 provided at the proximal end of the guide wire insertion channel 10 and led into the first tubular member 15. The guide wire inserted in the first tubular member 15 is led to the second tubular member 16 connected with the first tubular member 15 constituting the guide wire drawing-out guiding mechanism 14. The guide wire 9 led into the second tubular member 16 is drew out through the guide wire drawing-out opening 13 toward the side direction of the sheath 1.

After fixing, e.g. grasping the end of the guide wire 9 drew out toward the side direction of the sheath 1, the catheter 2 is inserted into the blood vessel with the guidance of the guide wire 9 to locate the balloon 3 attached on the distal end of the catheter 2 and the stent mounted on the balloon 3 at the stenosed portion which is the site for stent implantation. At this time, the catheter 2 is inserted into the blood vessel together with the sheath 1.

When the balloon 3 reaches the stenosed portion along with the stent 4, the clamp fixing mechanism 25 is operated to release the fixing between the sheath 1 and the catheter 2, and the sheath 1 is pulled with respect to the catheter 2 in the direction of arrow X1 shown in figure 9 such that the balloon 3 and the stent 4 is exposed out of the distal end of the sheath 1. This makes it possible for the balloon 3 to be inflated and for the stent 4 mounted on the balloon to be expanded in diameter.

When releasing the fixing with the clamp fixing mechanism 25 and then moving the sheath 1 against the catheter 2, the sheath 1 might be pushed toward the direction of arrow X2 shown in figure 2 against the catheter 2 due to a misoperation. In addition, due to an enforced insertion under a situation wherein the distal end of the catheter 2 abuts on an inner wall of a blood vessel, the sheath 1 might overpass the insertion guiding member 5 provided at the tip of the catheter 2 and move to in front of the catheter 2. If the sheath 1 overpasses the insertion guiding member 5 and move to in front of the catheter 2, the distal end 1a of the sheath 1 would be expanded in diameter by the insertion guiding member 5 and possibly scratch and injure an inner wall of the blood vessel.

In addition, if the distal end 1a of the sheath 1 protrudes from the tip of the catheter 2, the insertion guiding member 5 is engaged with the sheath 1, prohibiting smooth movement of the sheath 1 against the catheter 2. In this case, a problem would occur wherein the stent 4 held by the catheter 2 cannot be protruded from the sheath 1 and be implanted to a desired implantation site.

In the catheter apparatus of this embodiment, the first movement restrictor 35 prevents the distal end 1a of the sheath 1 from overpassing the insertion guiding member 5 and protruding from the distal end of the catheter 2. By this way, an operation in which the sheath 1 is pushed and moved to in front of the catheter 2 can be surely prevented, a safe insertion into a blood vessel is promised and the stent 4 on the balloon 3 can be surely implanted to a desired implantation site.

By pulling the sheath 1 with respect to the catheter 2 in order for the stent 4 on the balloon 3 to be exposed outwardly, the second tubular member 16 moves, along with the sheath 1, in the direction of arrow X1 shown in figure 7 such that the second tubular member 16 is pulled from the first tubular member 15. At this time, the first and second tubular members 15 and 16 are connected by insertion with the overlap length L2 which is longer than the migration length L1 sufficient to release the support of the distal end at which the balloon 3 is provided. Consequently, even when the sheath 1 is pulled with respect to the catheter 2 by the length L1 sufficient to release the support of the distal end at which the balloon 3 is provided and to expose the balloon 3 outwardly, the connection between the first and second tubular members 15 and 16 is maintained. Consequently, the first and second tubular members 15 and 16 are capable of keeping the connection therebetween at any time and guiding the guide wire 9 inserted therethrough.

After the balloon 3 protrudes from the distal end of the sheath 1, by using an indeflator, for example, an expansion medium is fed through the expansion medium supplying channel 7 provided in the catheter 2 and is supplied to the balloon 3 via the through hole 8 to inflate the balloon 3. As the balloon 3 is inflated, the stent 4 mounted on the balloon 3 is expanded in diameter. The stent 4 thus expanded in diameter comes to be in a state to scaffold the inner wall of the blood vessel from inside. Next, the expansion medium filling the balloon 3 is drawn through the expansion medium supplying channel 7 for decompression and contraction. The stent 4 previously expanded in diameter keeps the expanded state, is disengaged from the balloon 3 contracted in diameter and is remained at the stenting position in the blood vessel such that the implantation for keeping the state scaffolding the inner wall of the blood vessel is accomplished.

Furthermore, in the catheter apparatus of the present invention, when pulling the sheath 1 with respect to the catheter 2 in order for the stent 4 on the balloon 3 to be exposed outwardly, the second abutting stopper 41 of the sheath 1 abuts the other abutting surface 42 located at the proximal end of the movement restriction member 37 as shown in figure 10, thus restricting the movement of the sheath 1 against the catheter 2 in the direction of arrow X1 shown in figure 9. Consequently, protruding position of the stent 4 on the balloon 3 from the catheter 2 can be restricted correctly, making it possible to surely expose the stent 4 outwardly from the sheath 1 and to surely expand the stent 4 with inflation of the balloon 3. Furthermore, the connection status between the first and second tubular members 15 and 16 can be surely kept, and thus the insertion guiding with the guide wire 9 can be surely kept.

After implanting the stent 4 into the implantation site as explained above, the catheter 2 and the sheath 1 are pulled out of the blood vessel with the guidance of the guide wire 9. At this time, since the guide wire 9 is led out toward the side direction from the middle portion of the sheath 1 covering the catheter 2, removal operation can be quickly conducted without using an extensional guide wire. In other words, the catheter apparatus according to the present invention can achieve advantages of monorail catheters, while including the sheath 1 holding the stent 4 mounted on the balloon 3 provided on the catheter 2.

As explained hereinabove, the catheter apparatus according to the present invention effectively utilizes the advantages of the monorail catheter, surely prevents dislocation or disengagement of the stent 4 mounted on the balloon 3 provided on the catheter 2 and surely prevents injuring of a blood vessel such that the stent 4 can be surely implanted to a desired lesion site. Furthermore, the stent 4 can be quickly implanted in a vessel in a living body, such as a blood vessel.

Although the above described embodiment explains an example in which the present invention is applied to a monorail catheter, the present invention can also be applied to an over-the-wire catheter wherein a guide wire insertion hole is formed over entire length of a catheter from its distal end to its proximal end and, similarly to the above example, can prevent the distal end of the sheath 1 from overpassing the insertion guiding member 5 and protruding from the distal end of the catheter 2 such that protruding position of the stent 4 of the balloon 3 from the catheter 2 can be restricted correctly, injuring of a blood vessel can be surely prevented and the stent 4 can be surely implanted to a desired lesion site.

Furthermore, the present invention can be broadly applied to implantations of stents in a vessel of a living body such as a ureter and a bile duct rather than a blood vessel.

### Reference Signs List

1 sheath
2 catheter
3 balloon
4 stent
5 insertion
7 expansion medium supplying channel
9 guide wire
10 guide wire insertion channel
11 guide wire leading-out opening
13 guide wire drawing-out opening
14 guide wire drawing-out guiding mechanism
15 first tubular member
16 second tubular member
21 connecting member
22 catheter drawing-out port
23 indeflation port
25 clamp fixing mechanism
26 catheter compressing member
27 compressing member holder
28 compressing and fixing tool
33 compressing operation member
35 first movement restrictor
36 first abutting stopper
37 movement restriction member
40 second movement restrictor
41 second abutting stopper

## Claims

1. A medical catheter apparatus comprising:
a catheter having a distal end to which an insertion guiding member is attached, provided at the distal end with a balloon to be inflated by supplying an expansion medium to expand a vascular stent mounted on an outer periphery of the balloon, and provided with an expansion medium supplying channel extending from the proximal end to the distal end of the catheter for supplying the expansion medium to inflate the balloon and with a guide wire insertion channel extending at least from the distal end to the middle portion of the catheter for inserting a guide wire, and
a sheath into which the catheter is inserted, covering the outer periphery of the catheter from its distal side where the balloon is positioned through its proximal side, and being operated to move relative to the catheter between the section where it is covering the balloon attaching the vascular stent and the section where it is supposed to expose the vascular stent attached to the balloon, and
a first movement restrictor provided between the sheath and the catheter, protruding from the distal end of the catheter for restricting movement overpassing the insertion guiding member thereby preventing the sheath from protruding out from the distal end of the catheter.

2. The medical catheter apparatus according to claim 1, further comprising a connecting member, provided at a proximal portion of the sheath, having a catheter drawing-out port for drawing-out the catheter inserted into the sheath to the distal end of the sheath, and the first movement restrictor is provided inside the catheter drawing-out port.

3. The medical catheter apparatus according to claim 2, wherein the first movement restrictor comprises an abutting stopper formed inside the catheter drawing-out port and a movement restriction member provided at the catheter for abutting the abutting stopper to restrict relative movement between the sheath and the catheter.

4. The medical catheter apparatus according to any one of claims 1 to 3, wherein
the insertion guiding member includes a proximal portion having a diameter larger than an inner diameter of the sheath and is formed as a shape tapered off toward its distal end, and
the movement of the sheath against the catheter is restricted by the first movement restrictor, thereby preventing the distal portion of the sheath from overriding the insertion guiding member and being expanded in diameter.

5. The medical catheter apparatus according to any one of claims 1 to 4, further comprising a catheter fixing mechanism, provided at a proximal portion of the sheath, for clamping and supporting the sheath against the catheter, thereby fixing the movement of the sheath against the catheter.

6. The medical catheter apparatus according to any one of claims 1 to 5, further comprising
a guide wire leading-out opening provided at a middle portion of the catheter for leading out the guide wire inserted from the distal end of the catheter toward the side direction of the catheter,
a guide wire drawing-out opening provided at a middle portion of the sheath for drawing out the guide wire led out from the catheter out of the sheath, and
a guide wire drawing-out guiding mechanism provided between the guide wire leading-out opening and the guide wire drawing-out opening for connecting the guide wire leading-out opening and the guide wire drawing-out opening and for guiding the guide wire led out from the guide wire leading-out opening to the guide wire drawing-out opening, wherein
the guide wire drawing-out guiding mechanism moves in accordance with the relative movement between the sheath and the catheter, with its length extending from the guide wire leading-out opening to the guide wire drawing-out opening variable.

7. The medical catheter apparatus according to claim 6, wherein the guide wire drawing-out guiding mechanism comprises
a first tubular member, the proximal end of which is connected with the guide wire leading-out opening, extending along the outer surface of the catheter toward the guide wire drawing-out opening formed on the sheath, and
a second tubular member, the proximal end of which is connected with the guide wire drawing-out opening formed on the sheath 1, extending along the inner surface of the sheath toward the guide wire leading-out opening formed on the catheter, wherein
the first tubular member and the second tubular member are movably connected by fitting their end portions.

8. The medical catheter apparatus according to claim 7, wherein
the first tubular member constituting the guide wire drawing-out guiding mechanism is formed as a tubular member having a length sufficient to be inserted into the second tubular member by an overlap length L2 longer than a migration length L1 of the sheath to outwardly expose the balloon provided at the distal portion of the catheter when the sheath is pulled with respect to the catheter.

9. The medical catheter apparatus according to any one of claims 6 to 8, further comprising a second movement restrictor provided between the sheath and the catheter for restricting protruding-out length of the end portion of the catheter to which the balloon is attached.

10. The medical catheter apparatus according to claim 9, wherein the second movement restrictor comprises a stopping step formed in the catheter drawing-out port and an abutting stopper provided on an outer periphery of the catheter for abutting the stopping step to restrict relative movement between the sheath and the catheter.
